# EUROPEAN PATENT APPLICATION

(11) **EP 4 434 537 A1**
(43) Date of publication of application: **25.09.2024**
(21) Application number: 22893268.7
(22) Date of filing: 11.11.2022
(51) Int. Cl.: A61K 38/48, A61K 9/19, A61K 9/08, A61P 17/00, A61K 8/66, A61Q 19/00

(54) **BOTULINUM NEUROTOXIN COMPOSITION**

(30) Priority: 15.11.2021 KR 20210156686; 15.11.2021 KR 20210156703
(71) Applicant: Medytox Inc., Chungcheongbuk-do 28126 (KR)
(72) Inventor: KWAK, Seongsung, Suwon-si, Gyeonggi-do 16228 (KR); KWON, JinHee, Suwon-si, Gyeonggi-do 16509 (KR); BYUN, JaeYoon, Yongin-si Gyeonggi-do 17095 (KR)
(74) Representative: Brevalex
(86) International application number: PCT/KR2022/017751
(87) International publication number: WO 2023/085849

(57) **Abstract**

The present disclosure relates to a botulinum toxin composition which has an effect of reduced diffusion and/or an effect of extended duration.

## Description

### Technical Field

The present disclosure relates to a Botulinum neurotoxin composition.

### Background Art

Botulinum neurotoxin (BoNT) is a polypeptide product of the anaerobic bacterium *Clostridium Botulinum,* and it paralyzes muscles by preventing the secretion of neurotransmitters at the area where nerves and muscles meet. Botulinum toxin was originally a toxic substance that causes death, but after it was first used to treat diseases caused by excessive muscle contraction, including the treatment of strabismus, its scope of use has expanded and is now used for a variety of purposes. Botulinum toxin is mainly used to treat dystonia and other non-dystonia movement disorders such as hemifacial spasm, tremor, and spasticity. In addition, it is used for various indications, including ocular diseases, pain-related diseases such as migraine, urologic diseases, and wrinkle treatment for cosmetic purposes.

Korean Publication No. KR 2016-0113597 relates to a botulinum toxin composition that does not include animal protein, and use thereof in a patient in need of treatment for a disease, disorder, or condition, and discloses that a botulinum toxin composition that does not include animal protein has an extended effect of the botulinum toxin compared to a botulinum toxin composition that includes animal protein.

However, no botulinum toxin composition with a reduced diffusion effect has been known.

Meanwhile, Korean Publication Nos. KR 2008-0050636 and KR 2008-0049152 respectively disclose pharmaceutical compositions composed of botulinum toxin or a mixture of botulinum toxin without the complex protein naturally formed with botulinum toxin. In addition, Korean Publication No. KR 2016-0113597 relates to a botulinum toxin composition that does not include animal protein, and use thereof in a patient in need of treatment for a disease, disorder, or abnormality, and the botulinum toxin composition that does not include animal protein has an extended effect of botulinum toxin compared to a botulinum toxin composition that includes animal protein.

However, there has been no attempt to extend the effect of a botulinum toxin composition that includes a non-complex botulinum toxin as an active ingredient and free of animal protein.

### Disclosure

### Technical Problem

An object of the present disclosure is to provide a Botulinum toxin composition having a reduced diffusion effect, its use in treating a disease, or a method of treating a disease by administering it to a subject in need thereof.

An object of the present disclosure is to provide a Botulinum toxin composition including a non-complex Botulinum toxin as an active ingredient and free of animal protein, its use in preparing a medicament for treating a disease, or a method of treating a disease by administering it to a subject in need thereof.

### Technical Solution

The present disclosure relates to a Botulinum toxin composition having a reduced diffusion effect and/or an effect for an extended period of time.

### Advantageous Effects

According to an embodiment of the present disclosure, provided is a Botulinum toxin composition that has a reduced diffusion effect and thus decreases the risk of side effects due to diffusion, its use in preparing a medicament for treating diseases, and a treatment method using the same.

According to the present disclosure, provided is a Botulinum toxin composition including a non-complex Botulinum toxin as an active ingredient and free of animal protein, which has an extended effect and thus increases therapeutic effect and patient convenience, and its use in preparing a composition for treating a disease, and a treatment method using the same.

### Description of Drawings

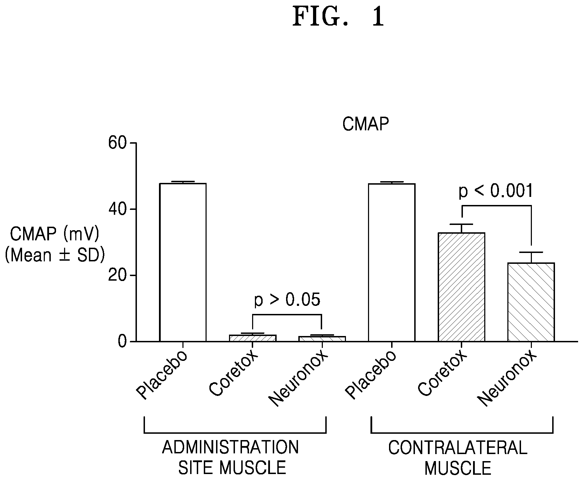

FIG. 1 is a graph illustrating CMAP values in the administered site muscle and contralateral non-administered muscle in mice on the 7th day after administering two types of botulinum toxin type A products (Coretox, Neuronox) and a placebo to the right gastrocnemius muscle, as described in Example 1 of the present specification. CMAP values were analyzed using a two-tailed t-test.

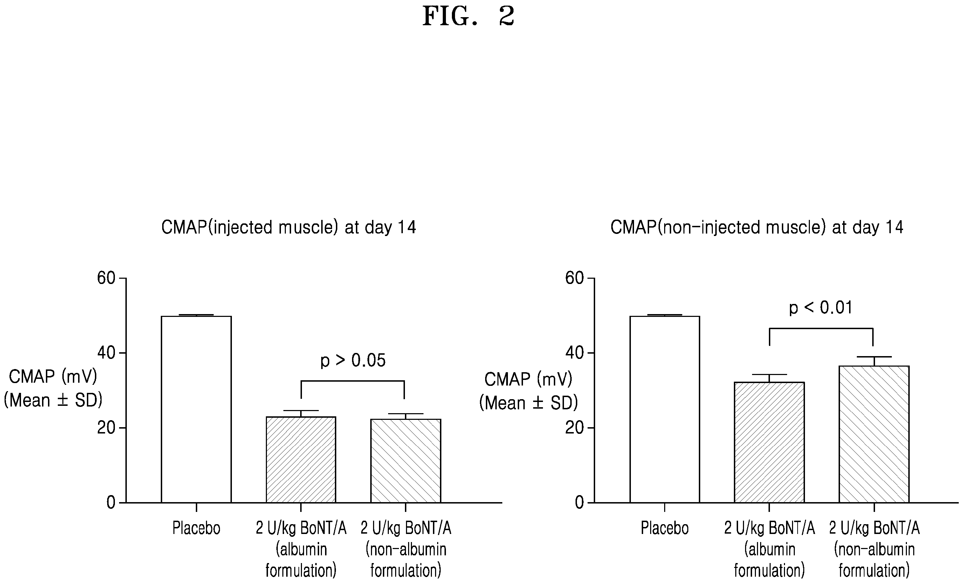

FIG. 2 shows CMAP values in the right anterior tibial muscle (administered muscle) and right gastrocnemius muscle (non-administered muscle) in rats, on the 14th day after administering two different formulations of complexed botulinum toxin type A (TFXA21004; albumin formulation, NXA20001; non-albumin formulation) and a placebo to the right tibialis anterior muscle, as described in Example 2 of the present specification. CMAP values were analyzed using a two-tailed t-test.

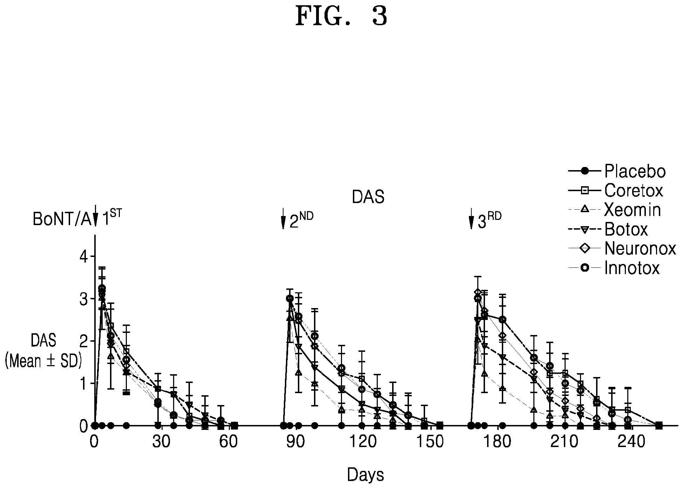

FIG. 3 shows a graph illustrating changes in DAS values over time in rats after three repeated administration of 5 types of botulinum toxin type A products (Coretox, Xeomin, Botox, Neuronox, Innotox) and a placebo to the right gastrocnemius muscle, as described in Example 3 of the present specification. The arrow at the top of the graph indicates the time when each botulinum toxin type A product was administered.

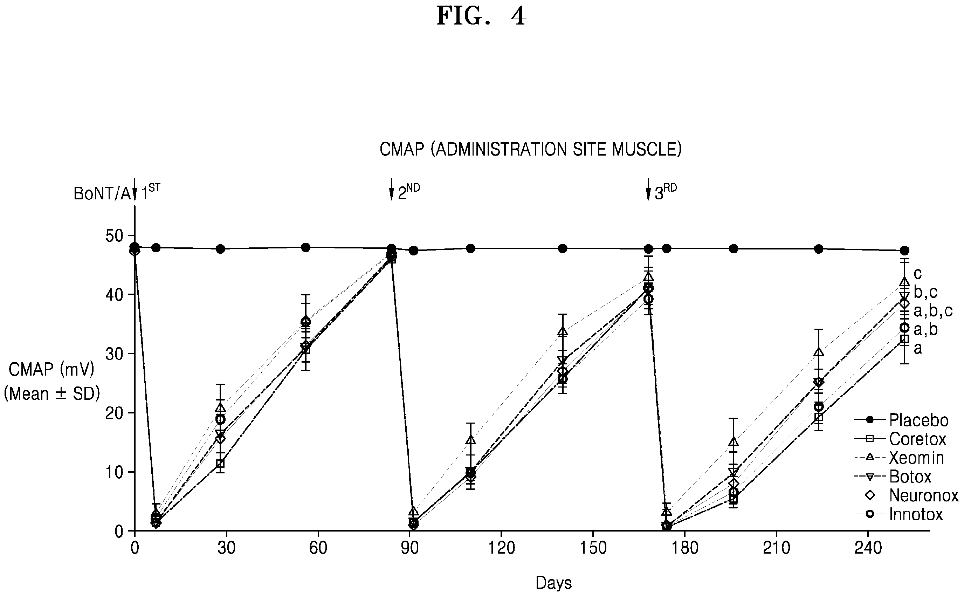

FIG. 4 shows a graph illustrating changes in CMAP values over time in mice after three repeated administration of 5 types of botulinum toxin type A products (Coretox, Xeomin, Botox, Neuronox, Innotox) and a placebo to the right gastrocnemius muscle, as described in Example 3 of the present specification. The arrow at the top of the graph indicates the time when each botulinum toxin type A product was administered, and at the end point (on 252nd day after the first administration), a one-way ANOVA was performed on the CMAP value of each botulinum otoxin administration group, and Tukey's post hoc test was used to compare between groups. ^{a-c} Each different letter represents a statistically significant difference.

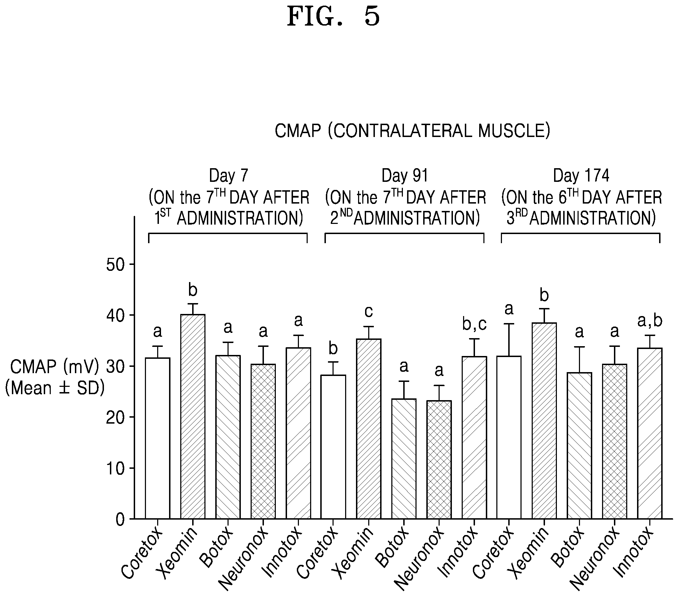

FIG. 5 shows a graph illustrating the CMAP levels of the contralateral side in mice, on the 6th to 7th day after administering 5 types of botulinum toxin type A products (Coretox, Xeomin, Botox, Neuronox, Innotox) and a placebo to the right gastrocnemius muscle three times, as described in Example 3 of the present specification. A one-way ANOVA was performed on the CMAP values of the contralateral muscle of each botulinum toxin administration group, and Tukey's post hoc test was used to compare between groups. ^{a-c} Each different letter represents a statistically significant difference.

### Mode for Invention

Unless otherwise defined, technical and scientific terms used herein have the same meanings as those commonly used in the field to which this disclosure belongs. For purposes of understanding this disclosure, the following definitions will apply and terms used in the singular include the plural and vice versa.

As used herein, the term 'Botulinum toxin' refers to a Botulinum toxin protein molecule produced by bacteria or recombination, including all serotypes, variants or fusion proteins thereof.

In an embodiment, the Botulinum toxin may be used without limitation to serotypes, and may be selected from the group consisting of types A (BoNT/A), B (BoNT/B), C (BoNT/C), D (BoNT/D), and E (BoNT/E). ), F (BoNT/F), G (BoNT/G), H (BoNT/H), X (BoNT/X), enterococcus spp. Botulinum toxin J (eBoNT/J) and mosaic Botulinum toxin and/or variants thereof. Examples of the mosaic toxin include BoNT/DC, BoNT/CD, and BoNT/FA. For example, type A may be used.

In an embodiment, the Botulinum toxin is derived from various BoNT/A subtypes such as A1, A2, A3, A4, A5, A6, A7, A9 and A10; BoNT/B subtypes such as B1, B2, B3, B4, B5, B6, B7, B8, Bnp and Bbv; BoNT/C subtypes such as C and CD; BoNT/D subtypes such as D and DC; BoNT/E subtypes such as E1, E2, E3, E4, E5, E6, E7, E8 and E9; BoNT/F subtypes such as F1, F2, F3, F4, F5, F6 and F7; and BoNT/G subtype such as subtype G. BoNT subtypes include chimeric BoNTs, such as BoNT/DC, BoNT/CD, and BoNT/FA.

The Botulinum toxin includes Botulinum toxin derivatives. The Botulinum toxin derivative may be a derivative including one or more chemical or functional modifications on a part or a part of the chain of a natural Botulinum toxin or a recombinant native Botulinum toxin having Botulinum toxin activity. For example, the Botulinum toxin derivative may be a modified toxin having one or more amino acids deleted, modified, or substituted. The Botulinum toxins may be recombinant peptides, fusion proteins, or hybrid toxins made, for example, from subunits or domains of different toxin serotypes. The Botulinum toxin may also be a part of an overall molecule with toxin activity, and may be used as a part of a combination or conjugated molecules thereof, such as a fusion protein.

The term 'complex protein' refers to hemagglutinin and nontoxic nonhemagglutinin (NTNH) that does not have hemagglutinin activity.

*Clostridium Botulinum* strains produce immunologically distinct toxins (type A to G). The molecular masses of type A to G neurotoxin (NTX) are approximately 150 kDa (7S). In culture media and foods with acidic conditions, NTX combines with non-toxic components to form a large complex called progenitor toxins (PTX). PTX may have molecular masses of 900 kDa (19S), 500 kDa (16S) and 300 kDa (12S). The 12S toxin is composed of NTX and a non-toxic component (NTNH) without hemagglutinin activity, while the 19S and 16S toxins are composed of NTX, NTNH, and hemagglutinin. Type A strains produce three types of toxin (19S, 16S and 12S), while type B, C and D strains produce 16S and 12S toxins. Under alkaline conditions, PTX dissociates into NTX and non-toxic components, 19S and 16S toxins dissociate into NTX and NTNH and non-toxic components of hemagglutinin, and 12S toxin dissociates into NTX and NTNH.

The Botulinum toxin composition of the present disclosure may be a complex Botulinum toxin composition including a complex protein. For example, the complex Botulinum toxin may include NTNH or hemagglutinin, in which case the molecular weight of the complex Botulinum toxin may be about 900 kDa.

The Botulinum toxin composition of the present disclosure may be a non-complex Botulinum toxin composition that does not include complex proteins. For example, the non-complex Botulinum toxin may not include NTNH or hemagglutinin, in which case the molecular weight of the non-complex Botulinum toxin may be about 150 kDa.

The term 'animal protein' refers to products of blood origin, blood incorporation or other animal origin.

The Botulinum toxin composition of the present disclosure may be a Botulinum toxin composition that does not include animal protein. For example, the Botulinum toxin composition of the present disclosure may not include a protein stabilizer derived from animals. In an embodiment, the Botulinum toxin composition of the present disclosure may not include a human serum albumin or a recombinant human albumin.

The term 'active ingredient' refers to a substance of a biologically or physiologically effective agent or composition. In particular, in the context of pharmaceutical preparations or compositions, the term 'active ingredient' refers to an ingredient substance that exhibits a targeted pharmaceutical effect. For example, the active ingredient of the present disclosure may be a Botulinum toxin without complex protein.

'Administration' or 'administering' refers to providing a pharmaceutical composition or active ingredient to a subject. The pharmaceutical composition may be administered via a variety of suitable routes.

In an aspect, administration of the pharmaceutical composition may be by transdermal, subcutaneous, or intramuscular administration. In an embodiment, this may be accomplished by topically administering the composition to a muscle or group of muscles. For example, reduction of brow furrows and skin wrinkles may be achieved by administering the composition transdermally or subcutaneously to the wrinkles. For example, the composition of an embodiment may be administered by subcutaneous or intramuscular injection. The composition according to an embodiment may be administered by subcutaneous or intramuscular injection through a prefilled syringe. The composition of an embodiment may be administered transdermally using a transdermal patch or microneedle.

'Pharmaceutical composition' means a preparation including an active ingredient. In addition to the active ingredient (e.g., Botulinum toxin), the 'pharmaceutical composition' may include at least one additional ingredient, such as albumin (human serum albumin or recombinant human albumin) and/or sodium chloride. The pharmaceutical composition is a preparation suitable for administration to a subject such as a mammal, including human. The pharmaceutical composition may be in liquid or lyophilized form. If the pharmaceutical composition is a lyophilized preparation, the lyophilized pharmaceutical composition may be re-dissolved and used, for example, using saline solution or water. It may be in solution form. The pharmaceutical composition may be a liquid or a solid. The pharmaceutical composition may be free of animal derived proteins and/or albumin.

'Treat', 'treating' or 'treatment' means an alleviation or reduction (including partial reduction, significant reduction, almost complete reduction and complete reduction), resolution or prevention (whether temporary or permanent) of a disease, disorder or condition so as to achieve the desired therapeutic effect, for example, by healing of injured or damaged tissue, or altering, changing, strengthening, improving, ameliorating and/or beautifying a pre-existing or recognized disease, disorder or condition. In the present disclosure, 'treatment' is a concept that includes prevention. 'Prevention' means delaying the onset of a disease, disorder or condition. It may be considered as a complete prevention if the onset of the disease, disorder or condition is delayed for a predetermined period of time.

In one aspect, 'treatment' means treatment of a disease, disorder, or medical condition in a patient, such as a mammal (particularly a human), including one or more of the following:
(a) preventing the occurrence of said disease, disorder, or medical condition, i.e. preventing recurrence of said disease or medical condition or prophylactic treatment of a patient pre-disposed to said disease or medical condition;
(b) ameliorating the disease, disorder, or medical condition, i.e., eliminating or regressing the disease, disorder, or medical condition in the patient, including antagonizing the effects of other therapeutic agents;
(c) suppressing the disease, disorder, or medical condition, i.e., slowing or arresting the development of the disease, disorder, or medical condition in the patient; or
(d) alleviating symptoms of said disease, disorder, or medical condition in the patient.

As used herein, the term 'unit', 'unit(s)', or 'U' refers to a LD50 dose, defined as the amount of Botulinum toxin that kills 50% of the mice that received Botulinum toxin injection, which is used interchangeably within a product.

In an embodiment, the therapeutically effective amount of Botulinum toxin is about 0.01 U/kg to about 100 U/kg, about 0.1 U/kg to about 100 U/kg, about 0.2 U/kg to about 100 U/kg, about 0.2 U/kg. kg to about 50 U/kg, about 0.2 U/kg to about 30 U/kg, about 0.2 U/kg to about 10 U/kg, about 0.2 U/kg to about 1 U/kg. In another embodiment, it may be about 1 U to about 10,000 U, about 1 U to about 5,000 U, about 1 U to about 2,500 U, about 1 U to about 1,000 U, about 1 U to about 500 U, about 1 U to about 300 U, about 1 U to about 200 U, about 10 U to about 200 U, about 10 U to about 100 U, or about 10 U to about 50 U, based on an adult weighing 60 kg.

According to an embodiment of the present disclosure, provided is a Botulinum toxin composition that does not include animal protein. The Botulinum toxin composition may include a pharmaceutically acceptable excipient or additive that is not derived from animal protein. The composition of an embodiment is a composition that does not include animal protein and includes a Botulinum toxin as an active ingredient for use in a method for treating a disease in a patient in need of treatment, wherein the method includes topically administering a therapeutically effective amount of the composition to a subject in need of treatment, and diffusion may be reduced at the administration site compared to a Botulinum toxin composition including animal protein.

In an embodiment, the Botulinum toxin may be a non-complex Botulinum toxin.

The composition of an embodiment may have an effect at the site of administration for an extended period of time than a Botulinum toxin composition including animal protein.

In an aspect, the pharmaceutically acceptable excipient or additive is a stabilizer, an ionic compound, a surfactant, a buffer, a lyophilization protectant, or a combination thereof, such as amino acid (e.g., methionine), salt (e.g. NaCl), buffer, nonionic surfactant (e.g. polysorbates, e.g. polysorbate 20), sugar (e.g. disaccharides such as white sugar, etc.), sugar alcohol (e.g. sorbitol), or a combination thereof.

The Botulinum toxin composition of the present disclosure may be formulated in any form, such as solid or liquid formulation, for example, lyophilized powder, liquid, or pre-filled syringe formulation.

The composition of an embodiment may be a lyophilized composition. In an embodiment, the lyophilized composition includes a Botulinum toxin, a nonionic surfactant such as polysorbate, and/or an amino acid; it may include one or more components selected from the group consisting of a sugar, a sugar alcohol, and an ionic compound.

In an embodiment, the polysorbate may include, for example, polysorbate 20, 40, 60, 80, or 100, and may include, for example, polysorbate 20. In an embodiment, the amino acid may be methionine or isoleucine. For example, the liquid composition of an embodiment may include polysorbate 20 and methionine as stabilizers.

In an embodiment, the sugar may include trehalose, sucrose, maltose, fluctose, raffinose, lactose, or glucose. The sugar alcohols may include cyclodextrin, mannitol, sorbitol, glycerol, xylitol, or inositol. For example, the lyophilized composition of an embodiment may include white sugar (sucrose).

In an embodiment, the ionic compound may include sodium chloride, sodium phosphate, ammonium phosphate, magnesium sulfate, sodium acetate, sodium lactate, sodium succinate, sodium propionate, or potassium phosphate.

In the lyophilized composition of an embodiment, 0.01 mg to 2 mg of polysorbate, 0.01 mg to 10 mg of methionine, 0.1 mg to 50 mg of sugar, 0.1 mg to 50 mg of sugar alcohol, and 0.1 mg to 10 mg of ionic compound may be included, per 100 units of Botulinum toxin.

The composition of an embodiment may be a liquid composition. The liquid composition of an embodiment may include nonionic surfactants such as Botulinum toxin and polysorbate and/or amino acids. In an embodiment, the polysorbate may include, for example, polysorbate 20, 40, 60, 80, or 100, and may include, for example, polysorbate 20. In an embodiment, the amino acid may be methionine or isoleucine. For example, the liquid composition of an embodiment may include polysorbate 20 and methionine as stabilizer. In the liquid composition of an embodiment, the concentration of polysorbate may be 0.05 mg/mL to 2.5 mg/mL or 0.1 mg/mL to 2.5 mg/mL, and the concentration of methionine may be 0.5 mM to 50 mM or 25 mM to 75mM. The pH of the liquid composition in an embodiment may be 5.5 to 7.0.

The reduced diffusion effect or the effect for an extended period of time of the composition of an embodiment may occur upon repeated administration of one or more times, two or more times, three or more times, four or more times, or five or more times. The measurement value of the compound muscle action potential of the muscle adjacent to the muscle at the administration site of the composition of an embodiment may be higher than the measurement value of the compound muscle action potential of the muscle adjacent to the administration site when the same dose of the animal protein-including composition is administered at the same administration site and location. In the present disclosure, "muscle adjacent to the muscle at the administration site" refers to a muscle that is separated by a predetermined distance from the muscle at the administration site. In addition, "muscle adjacent to the administration site" may be a site that is preferably not affected (pharmacological action) by the composition of an embodiment.

The measurement value of the compound muscle action potential of the muscle at the administration site of the composition of an embodiment may be lower than the measurement value of the compound muscle action potential of the muscle at the administration site when the same dose of the animal protein-including composition is administered at the same administration site and location.

According to an embodiment of the present disclosure, provided may be a Botulinum toxin composition including non-complex Botulinum toxin as an active ingredient and not including animal protein for use in a method for treating a disease in a patient in need of treatment, wherein the method includes topically administering a therapeutically effective amount of the composition to a subject in need of treatment, and the Botulinum toxin composition has an effect at the site of administration for an extended period of time than a Botulinum toxin composition including animal protein.

Unless there is a contradiction, the parts described with respect to the Botulinum toxin composition in the present specification may be of course equally applied to a treatment method using the Botulinum toxin composition and to a use of the Botulinum toxin composition in the manufacture of medicament for treating a disease.

According to an embodiment of the present disclosure, provided is a use of a Botulinum toxin composition that does not include animal protein for improving or treating a disease by administering the Botulinum toxin composition into an individual.

The use of an embodiment is for improving skin wrinkles, square jaw, pointed chin, wounds, skin softening, scars, acne, pores, elasticity or keloids, or for treating facial spasms, blepharospasm, torticollis, blepharospasm, cervical dystonia, central pharyngeal dystonia, spasmodic dysphonia, migraine, anal pruritus, or hyperhidrosis, by administering a therapeutically effective amount of a composition including Botulinum toxin and a pharmaceutically acceptable excipient or additive to an individual.

According to another aspect of the present disclosure, provided is a method for improving or treating a disease by administering a composition including the Botulinum toxin and a pharmaceutically acceptable excipient or additive to an individual. The method of an embodiment may be a method for improving skin wrinkles, square jaw, pointed chin, wounds, skin softening, scars, acne, pores, elasticity, or keloids, or for treating facial spasms, blepharospasm, torticollis, blepharospasm, cervical dystonia, central pharyngeal dystonia, spasmodic dysphonia, migraine, anal pruritus, or hyperhidrosis, by administering a composition including the Botulinum toxin as an active ingredient and a pharmaceutically acceptable excipient or additive, and not including animal protein to an individual. In an embodiment, the skin wrinkles may include glabellar lines, brow furrows (e.g., horizontal wrinkles on the forehead), mouth lines, eye lines (e.g., lateral canthal line (fine lines around the eyes), under-eye lines), marionette lines, or lines on the bridge of the nose.

In an aspect, the Botulinum toxin may be administered in a single or multiple treatment sessions. The dosage may be administered as a single or divided injection at the injection site. In multiple treatment sessions, the Botulinum toxin may be administered at intervals of no more than 1 year, no more than 10 months, no more than 8 months, no more than 6 months, no more than 5 months, no more than 4 months, or no more than 3 months. For example, the administration interval may be 3 months or more and 1 year or less, 3 months or more and 6 months or less, or 4 months or more and 6 months or less. In multiple treatment sessions, the administration interval of the Botulinum toxin includes primary treatment and secondary treatment, and the dosage of the secondary treatment may be less than, more than, or the same as the dosage of the primary treatment.

The administered dose, administration time, administration method, administration period or interval, and the like of the Botulinum toxin may be appropriately selected and used by a skilled person in the art depending on the patient's weight, age, gender, health condition, severity of disease, and the like.

The subjects to which the Botulinum toxin or a composition including it is administered may include humans or animals, such as humans, pigs, dogs, cats, cows, horses, rats, and the like, without limitation.

According to an embodiment of the present disclosure, provided may be a use of the Botulinum toxin composition of claim 1, 2 or 12 in the preparation of a medicament for improving skin wrinkles, square jaw, pointed chin, wounds, skin softening, scars, acne, pores, elasticity, or keloids, or for treating facial spasms, blepharospasm, torticollis, blepharospasm, cervical dystonia, central pharyngeal dystonia, spasmodic dysphonia, migraines, anal pruritus, or hyperhidrosis.

According to another aspect of the present disclosure, provided is a method of preparing a Botulinum toxin composition including administering a composition including the Botulinum toxin as an active ingredient and a pharmaceutically acceptable excipient or additive to an individual, thereby exhibiting a reduced diffusion at the administration site compared to a Botulinum toxin composition including animal protein.

Hereinafter, the present disclosure will be described in more detail through examples, but this is only for illustrating the present disclosure and is not intended to limit its scope in any way.

### 1. Diffusion reduction effect of the composition according to an embodiment

### Example 1: Non-clinical efficacy test of Coretox and Neuronox in mice

Female 5-week-old CD1 (ICR) mice were purchased from Orient Bio Co., Ltd., and after acclimatization and quarantine for 1 week, 6-week-old mice were used in the experiment. The feed was supplied ad libitum with sterilized solid feed for laboratory animals (R40-10, SAFE, France), and drinking water was supplied ad libitum with autoclaved tap water. During the acclimation, quarantine, and experimental period, mice were housed under specific-pathogen-free conditions set at a temperature of 23 ± 3 °C, relative humidity of 55 ± 15 %, illumination time of 12 hours (8:00 a.m. to 8:00 p.m.), ventilation frequency of 15 times/hour, and illumination intensity of 150 to 300 Lux. This study was conducted after review and approval by the Medytox Animal Experiment Ethics Committee (A-2020-016).

The test material was prepared as follows. Coretox (Lot No: NSA20011, Medytox) and Neuronox (Neuronox, Lot No: TFAA20024, Medytox) were used (Table 1). For the placebo (placebo administration group), sterile saline solution (Daehan Pharmaceutical Industry) was used. Each test substance was diluted to a concentration of 60 U/ml using sterile saline solution.

**[Table 1]**

| Product name | Manufacturing company | Botulinum toxin | Additive |
|---|---|---|---|
| Coretox | Medytox | Approximately 150 kDa, non-complex Botulinum toxin type A | Methionine, Polysorbate 20, white sugar, sodium chloride |
| Neuronox | Medytox | About 900 kDa, Complex Botulinum toxin type A | Human serum albumin, sodium chloride |

The day on which the test substance was administered was set as day 0, and after anesthetizing the mouse using an injection anesthetic (100 mg/kg ketamine hydrochloride + 10 mg/kg xylazine), each test substance was administered at 0.2 mL/kg into the mouse's right gastrocnemius muscle using a Hamilton syringe according to Table 2.

The compound muscle action potential (CMAP) test, which measures the action potential of muscles that respond to external electrical stimulation, was used for evaluation. CMAP was measured at the right gastrocnemius muscle area of each mouse using a Nicolet Viking Quest (Viasys Healthcare, Inc.) equipment. After anesthesia with an injection anesthetic, fur was removed from the measurement area and the mouse was placed in a prone position. The cathode was placed on the sciatic nerve area on the side of the leg to be measured, and the anode was placed approximately 1 cm away from the area based on the spine. The recording electrode and reference electrode were placed on the gastrocnemius muscle region and Achilles tendon region, respectively, and the ground electrode was placed on the rectus femoris region. The level and duration of stimulation were 7 mA to 8 mA, 0.1 ms, and the filter range of the amplifier was set to 2 K to 10 K at 60 Hz. When measured under the relevant conditions, the height from the base of the waveform to the peak was converted into CMAP measurement data. On the 7th day after administration of the test substance, measurements were performed on the gastrocnemius muscle at the injection site and the gastrocnemius muscle of the opposite leg.

Graphpad Prism 7.05 (GraphPad Software Inc., CA, USA) was used for graph presentation, and SPSS software 25.0 (SPSS Inc., IL, USA) and Excel (2013, MS, USA) were used for statistical analysis. The results of the experiment were expressed as mean ± standard deviation. Normality was tested using the Kolmogorov-Smirnov test. Non-parametric data were statistically analyzed using the Mann-Whitney test, and for parametric data, a two-tailed t-test was conducted, and if the p-value was less than 0.05, it was judged to be statistically significant.

**[Table 2]**

| Group | Number of animals | Administered substance | Substance information | Capacity (U/kg) | Admin istration route | Evaluation indicator |
|---|---|---|---|---|---|---|
| 1 | 6 | Placebo | Sterile saline solution | 0 | Right gastrocnemius muscle | CMAP |
| 2 | 6 | Coretox | Non-albumin formulation | 12 | Right gastrocnemius muscle | CMAP |
| 3 | 6 | Neuronox | Albumin formulation | 12 | Right gastrocnemius muscle | CMAP |

On the 7th day after administration of two types of Botulinum toxin type A products (Coretox, Neuronox) at a dose of 12 U/kg to the right gastrocnemius muscle of the mouse, CMAP measurements were performed on the muscle at the administered site and the muscle at the non-administered site on the opposite side. As a result, no significant difference was observed in the CMAP value of the muscle at the injection site between the Coretox and Neuronox administration groups, but the CMAP value of the contralateral muscle was observed to be significantly higher in the Coretox administration group compared to the Neuronox administration group (FIG. 1). From these results, the effect of reducing the diffusion of Botulinum toxin in the non-albumin formulation Coretox administration group was confirmed compared to the albumin formulation Neuronox administration group.

### Example 2: Non-clinical efficacy testing of different formulations of Botulinum toxin type A in rats

It was confirmed through the experiment below that the diffusion reduction effect of this non-albumin formulation was the same even when a 900 kDa toxin was used.

Female 6-week-old SD rat mice were purchased from Orient Bio Co., Ltd., and after acclimatization and quarantine for 1 week, 7-week-old rats were used in the experiment. The feed was supplied ad libitum with sterilized solid feed for laboratory animals (R40-10, SAFE, France), and drinking water was supplied ad libitum with autoclaved tap water. During the acclimation, quarantine, and experimental period, mice were housed under specific-pathogen-free conditions set at a temperature of 23 ± 3 °C, relative humidity of 55 ± 15 %, illumination time of 12 hours (8:00 a.m. to 8:00 p.m.), ventilation frequency of 15 times/hour, and illumination intensity of 150 to 300 Lux. This study was conducted after review and approval by the Medytox Animal Experiment Ethics Committee (A-2020-016).

The test material was prepared as follows. TFXA21004 (Medytox) and NXA20001 (Medytox), which are finished products of Botulinum toxin type A complexed in units of 100 units, were used (Table 3). For the placebo (placebo administration group), sterile saline solution (Daehan Pharmaceutical Industry) was used. Each test substance was diluted to a concentration of 100 U/mL using sterile physiological saline.

**[Table 3]**

| Test substance name | Manufacturi ng company | Botulinum toxin | Additive |
|---|---|---|---|
| NXA20001 | Medytox | About 900 kDa, Complex Botulinum toxin type A | Methionine, Polysorbate 20, white sugar, sodium chloride |
| TFXA2100 4 | Medytox | About 900 kDa, Complex Botulinum toxin type A | Human serum albumin, sodium chloride |

The day on which the test substance was administered was set as day 0. After anesthetizing the rats using an injection anesthetic (60 mg/kg ketamine hydrochloride + 10 mg/kg xylazine), each test substance was administered at 0.02 mL/kg to the rat's right tibialis anterior muscle using a Hamilton syringe according to the group composition (Table 4).

**[Table 4]**

| Group | Number of animals | Administered substance | Substance information | Capacity (U/kg) | Administration route | Evaluation indicator |
|---|---|---|---|---|---|---|
| 1 | 6 | Placebo | Sterile saline solution | 0 | Right anterior tibialis muscle | CMAP |
| 2 | 6 | TFXA21004 | Albumin formulation | 2 | Right anterior tibialis muscle | CMAP |
| 3 | 6 | NXA20001 | Non-albumin formulation | 2 | Right anterior tibialis muscle | CMAP |

The compound muscle action potential (CMAP) test was used for evaluation. The CMAP was measured at the right anterior tibial muscle (administered muscle) and gastrocnemius muscle (non-administered muscle) of each rat on 14th day after administration of the test substance using a UltraPro S100 (Natus neurology Inc.) equipment. After anesthesia with an injection anesthetic, fur was removed from the measurement area and the mouse was placed in a prone position. The anode was placed on the right psoas muscle between the 4th and 5th lumbar vertebrae of the rat, and the cathode was placed on the right sciatic nerve based on the 5th and 6th lumbar vertebrae. The distance between the anode and cathode was maintained at 1.5 cm. The recording electrodes were placed on the tibialis anterior muscle and gastrocnemius muscle, the reference electrode was placed on the Achilles tendon, and the ground electrode was placed on the rectus femoris muscle. The level and duration of stimulation were 20 mA to 30 mA, 0.2 ms, and the filter range of the amplifier was set to 2 K to 10 K at 60 Hz. When measured under the relevant conditions, the height from the base of the waveform to the peak after the latency was converted into CMAP measurement data. The obtained data were statistically processed in the same manner as in Example 1.

On the 14th day after administering two different formulations of complex Botulinum toxin type A (TFXA21004; albumin formulation, NXA20001; non-albumin formulation) to the right anterior tibial muscle of a rat at a dose of 2 U/kg, CMAP measurements were performed on the right anterior tibial muscle (administrated muscle) and the right gastrocnemius muscle (non-administered muscle). As a result, no difference in CMAP values between the two test substances was observed in the right tibialis anterior muscle, but a significantly increased CMAP value was observed in the NXA20001-administered group compared to the TFXA21004-administered group in the right gastrocnemius muscle, a non-administered muscle (FIG. 2). From these results, it was confirmed that the non-animal complexed Botulinum toxin type A formulation was effective in reducing the diffusion of Botulinum toxin into adjacent muscles compared to the animal formulation.

### Example 3: Non-clinical efficacy test of five Botulinum toxin products in mice

Female 5-week-old CD1 (ICR) mice were purchased from Orient Bio, and after acclimatization and quarantine for 1 week, 6-week-old mice were used in the experiment. The feed was supplied ad libitum with sterilized solid feed for laboratory animals (R40-10, SAFE, France), and drinking water was supplied ad libitum with autoclaved tap water. During the acclimation, quarantine, and experimental period, mice were housed under specific-pathogen-free conditions set at a temperature of 23 ± 3 °C, relative humidity of 55 ± 15 %, illumination time of 12 hours (8:00 a.m. to 8:00 p.m.), ventilation frequency of 15 times/hour, and illumination intensity of 150 to 300 Lux. This study was conducted after review and approval by the Medytox Animal Experiment Ethics Committee (A-2020-016).

The test substance was prepared as follows. Coretox (Lot No: NSA20011, Medytox), Neuronox (Lot No: TFAA20024, Medytox), Innotox (Lot No: D020003, Medytox), Xeomin (Lot No: 925559, Merz),and Botox (Lot No: C5394C3, Allergan) were used (Table 5). For the placebo (placebo administration group), sterile saline solution (Daehan Pharmaceutical Industry) was used. Each test substance was diluted to a concentration of 60 U/ml using sterilized saline, and Innotox was used as a liquid product.

**[Table 5]**

| Product name | Manufacturin g company | Botulinum toxin | Additive |
|---|---|---|---|
| Coretox | Medytox | About 150 kDa, non-complex Botulinum toxin type A | Methionine, Polysorbate 20, white sugar, sodium chloride |
| Neuronox | Medytox | About 900 kDa, Complex Botulinum toxin type | Human serum albumin, sodium chloride |
| Innotox | Medytox | About 900 kDa, Complex Botulinum toxin type | Methionine, Polysorbate 20, Sodium chloride |
| Xeomin | Multz | About 150 kDa, non-complex Botulinum toxin type A | Human serum albumin, white sugar |
| Botox | Allergan | About 900 kDa, Complex Botulinum toxin type | Human serum albumin, sodium chloride |

The day on which the test substance was administered was set as day 0, and after anesthetizing the mouse using an injection anesthetic (100 mg/kg ketamine hydrochloride + 10 mg/kg xylazine), Corenox, Neuronox, Xeomin, and Botox were administered at 0.2 mL/kg, and Innotox was administered at 0.3 mL/kg into the mouse's right gastrocnemius muscle using a Hamilton syringe according to Table 6. After the first administration, the second and third test substances were prepared in the same way as the first administration at an interval of 84 days and administered at the same location and in the same dose.

The evaluation includes the digit abduction score (DAS) evaluation method, which visually evaluates the degree of mouse muscle paralysis, and the compound muscle action potential (CMAP) test, which measures the action potential of muscles responding to external electrical stimulation. The DAS value shown in Table 7 represents the degree of muscle paralysis according to the shape of the toe on the administered side in the mouse, and the CMAP value represents the degree of inhibition of direct muscle contraction caused by nerve block. The DAS recovery period refers to the period during which the DAS score returns to 0 after test substance administration. CMAP measurement was conducted in the same manner as in Example 1.

Graphpad Prism 7.05 (GraphPad Software Inc., CA, USA) was used for graph presentation, and SPSS software 25.0 (SPSS Inc., IL, USA) and Excel (2013, MS, USA) were used for statistical analysis. The results of the experiment were expressed as mean ± standard deviation. AUC (area under the curve) and AAC (area above the curve) values in DAS and CMAP graphs were calculated using Graphpad Prism software. One-way ANOVA was performed, and Tukey's post-hoc test was performed for comparison between groups. During analysis, if the p-value was less than 0.05, it was judged to be statistically significant.

**[Table 6]**

| Group | Number of animals | Administered substance | Substance information | dose (U/kg) | Administration route | Evaluation indicator |
|---|---|---|---|---|---|---|
| 1 | 8 | Placebo | Sterile saline solution | 0 | Right gastrocnemius muscle | DAS, CMAP |
| 2 | 8 | Coretox | Non-albumin formulation | 12 | Right gastrocnemius muscle | DAS, CMAP |
| 3 | 8 | Xeomin | Albumin formulation | 12 | Right gastrocnemius muscle | DAS, CMAP |
| 4 | 8 | Botox | Albumin formulation | 12 | Right gastrocnemius muscle | DAS, CMAP |
| 5 | 8 | Neuronox | Albumin formulation | 12 | Right gastrocnemius muscle | DAS, CMAP |
| 6 | 8 | Innotox | Non-albumin formulation, liquid | 12 | Right gastrocnemius muscle | DAS, CMAP |

**[Table 7]**

| | Mouse DAS evaluation criteria |
|---|---|
| Score | Criteria |
| 0 | Normal, no different from the shape of the foot on the non-injected side |
| 1 | The space between the toes is narrowed, or two toes are stuck together and the rest are fully extended |
| 2 | All toes are significantly narrowed, or all three toes are stuck together |
| 3 | Foot is curved and the four toes are stuck together. |
| 4 | Foot is curved and all toes are stuck together |

After administering 5 types of Botulinum toxin type A products (Coretox, Xeomin, Botox, Neuronox, Innotox) to the mouse's right gastrocnemius muscle at a dose of 12 U/kg in three times with an interval of 84 days, DAS evaluation shows that although no significant difference in the DAS recovery was observed after the first administration, a significant increase in the DAS recovery period was observed in the Coretox and Innotox administered groups compared to the Xeomin administered group after the second and third administration. In addition, after the third administration, a significant increase in the DAS recovery period was observed in the Coretox administration group compared to the Botox and Neuronox administration groups (Table 8). In the DAS graph, after the third administration on day 168, recovery was observed for all subjects on day 252 in the Coretox and Innotox administration groups, recovery was observed for all subjects on day 231 in the Botox and Neuronox administration groups, and recovery was observed for all subjects on day 217 for Xeomin, thus a long duration of action was observed in the Coretox and Innotox administered groups (FIG. 3). Similarly, DAS_{AUC}, which is an indicator of cumulative pharmacological effect over time, was observed to be significantly higher in the Coretox, Innotox, and Neuronox administration groups compared to the Xeomin administration group after the second and third administration, and DAS_{AUC} was observed to be significantly higher in the Coretox and Innotox administration group compared to the Botox administration group after the third administration (Table 8).

Similar to the DAS evaluation, in the CMAP measurement, the CMAP measurement of the muscle at the injection site on day 252, which is the end point after the third administration on day 168, showed significantly lower CMAP values in the Coretox and Innotox administration groups compared to the Xeomin administration group, showing strong efficacy (Figure 4). In addition, as a result of CMAP_{AAC} comparison, after the first administration, the CMAP_{AAC} of the Coretox, Neuronox, and Botox administration groups was observed to be significantly higher than the Xeomin administration group, and the CMAP_{AAc} of the Coretox administration group was observed to be significantly higher than the Innotox administration group. After the second administration, the CMAP_{AAC} of the Coretox, Neuronox, Botox, and Innotox administration groups was observed to be significantly higher than the Xeomin administration group, and after the third administration, the CMAP_{AAc} of the Coretox administration group was observed to be significantly higher than the Xeomin administration group (Table 9). Referring to FIG. 5, as a result of CMAP measurement of the contralateral muscle on 6th to 7th day after each Botulinum toxin administration, a significant increase in the CMAP value of the contralateral muscle was observed in the Coretox and Innotox administered groups compared to the Botox and Neuronox administered groups after the second administration. As it was confirmed that Xeomin has a weaker effect compared to the four types of Botulinum toxin (see FIG. 4), the significant increase in the CMAP value of the contralateral muscle in the Xeomin administration group is believed to be not due to the anti-diffusion effect, but to its weaker effect compared to the four types of Botulinum toxin products.

From these results, the effects of three repeated administrations of five types of Botulinum toxin type A products were compared in mice, and as a result, non-albumin-type Botulinum toxin (Coretox, Innotox) was confirmed to have stronger efficacy, increased duration of action, and reduced diffusion effect compared to albumin-type Botulinum toxin products (Xeomin, Botox, Neuronox).

**[Table 8]**

| DAS recovery period and DAS_{AUC} results | | | | | | |
|---|---|---|---|---|---|---|
| | DAS recovery period (days) | | | DAS_{AUC} (score·day) | | |
| product | After 1st administrat ion (day 0-84) | After 2nd administrati on (day 84-168) | After 3rd administrati on (day 168-252) | After 1st administrat ion (day 0-84) | After 2nd administrati on (day 84-168) | After 3rd administrati on (day 168-252) |
| Coretox | 42.0±8.4^{a} | 52.5±11.2^{b} | 68.3±13.4^{c} | 59.4±11.5^{a} | 75.6±27.6^{b} | 103.4±27.4^{c} |
| Xeomin | 35.0±8.4^{a} | 33.1±14.3^{a} | 32.4±11.8^{a} | 40.4±16.4^{a} | 34.7±17.2^{a} | 29.9±16.0^{a} |
| Botox | 45.4±11.0^{a} | 41.8±11.0^{a,b} | 44.6±10.5^{a,b} | 55.1±13.8^{a} | 51.0±17.1^{a,b} | 57.1±23.2^{a,b} |
| Neurono x | 36.6±11.4^{a} | 50.0±10.2^{a,b} | 49.0±11.1^{a,b} | 49.7±19.7^{a} | 71.6±18.2^{b} | 68.2±16.7^{b,c} |
| Innotox | 34.1±7.9^{a} | 50.8±11.7^{b} | 62.0±13.1^{b,c} | 43.8±11.5^{a} | 75.7±24.7^{b} | 98.4±28.3^{c} |

| | | | | | | |
|---|---|---|---|---|---|---|
| ^{a-c} Different superscripts indicate statistically significant differences (one-way ANOVA, Tukey's post-hoc test) | | | | | | |

**[Table 9]**

| CMAP_{AAC} Results | | | |
|---|---|---|---|
| | CMAP_{AAc} (mV·day) | | |
| product | After 1st administration (day 0-84) | After 2nd administration (day 84-168) | After 3rd administration (day 168-252) |
| Coretox | 2045.2±169.4^{c} | 2143.1±134.5^{b} | 2181.5±262.0^{b} |
| Xeomin | 1611.1±264.0^{a} | 1783.7±126.4^{a} | 1629.6±187.9^{a} |
| Botox | 1918.4±108.7^{b,c} | 2075.8±199.3^{b} | 1750.3±242.5^{a} |
| Neuronox | 1912.2±159.5^{b,c} | 2129.0±169.3^{b} | 1784.4±218.1^{a} |
| Innotox | 1667.2±135.0^{a,b} | 2238.5±149.0^{b} | 1936.2±135.5^{a,b} |

| | | | |
|---|---|---|---|
| ^{a-c} Different superscripts indicate statistically significant differences (one-way ANOVA, Tukey's post-hoc test) | | | |

## Claims

1. A botulinum toxin composition comprising a botulinum toxin as an active ingredient and free of animal protein, for use in a method for treating a disease in a patient in need of treatment,
wherein the method comprises topically administering a therapeutically effective amount of the composition to a subject in need of treatment, and
at an administration site, diffusion is reduced compared to a botulinum toxin composition comprising animal protein.

2. The botulinum toxin composition of claim 1, wherein the botulinum toxin is a non-complex botulinum toxin.

3. The botulinum toxin composition of claim 1 or 2,
wherein the composition has an effect at the administration site for a longer period of time than a botulinum toxin composition comprising animal protein.

4. The botulinum toxin composition of claim 1 or 2, wherein the composition is a lyophilized composition.

5. The botulinum toxin composition of claim 4, wherein the lyophilized composition comprises a botulinum toxin, polysorbate, methionine; and one or more components selected from the group consisting of sugars, sugar alcohols, and ionic compounds.

6. The botulinum toxin composition of claim 1 or 2, wherein the botulinum toxin composition free of animal protein is a liquid composition.

7. The botulinum toxin composition of claim 5, wherein the liquid composition comprises a botulinum toxin, polysorbate, and methionine.

8. The botulinum toxin composition of any one of claims 1 to 7, wherein the composition is for improving skin wrinkles, square jaw, pointed chin, wounds, skin softening, scars, acne, pores, elasticity or keloid symptoms, or for treating facial spasms, blepharospasm, torticollis, blepharospasm, cervical dystonia, central pharyngeal dystonia, spasmodic dysphonia, migraine, anal pruritus, or hyperhidrosis.

9. The botulinum toxin composition of claim 1 or 3, wherein a reduced diffusion effect or an effect of extended duration occurs upon two or more repeated administrations of the botulinum toxin composition.

10. The botulinum toxin composition of claim 1 or 2,
wherein a measurement value of a compound muscle action potential of a muscle adjacent to a muscle at the administration site of the composition is higher than a measurement value of the compound muscle action potential of the muscle adjacent to the administration site when a same dose of an animal protein-containing composition is administered at the same administration site and location.

11. The botulinum toxin composition of any one of claims 1 to 3,
wherein a measurement value of a compound muscle action potential of a muscle at the administration has a lower value than a measurement value of the compound muscle action potential of the muscle at the administration site when a same dose of an animal protein-containing composition is administered at the same administration site and location.

12. A composition comprising a non-complex botulinum toxin as an active ingredient and free of animal protein, for use in a method for treating a disease in a patient in need of treatment,
wherein the method comprises topically administering a therapeutically effective amount of the composition to a subject in need of treatment, and
the composition has an effect at an administration site for a longer period of time than a botulinum toxin composition comprising animal protein.

13. A use of the botulinum toxin composition of claim 1, 2 or 12 in preparation of a medicament for improving skin wrinkles, square jaw, pointed chin, wounds, skin softening, scars, acne, pores, elasticity or keloid symptoms, or for treating facial spasms, blepharospasm, torticollis, blepharospasm, cervical dystonia, central pharyngeal dystonia, spasmodic dysphonia, migraine, anal pruritus, or hyperhidrosis.

14. A method for improving skin wrinkles, square jaw, pointed chin, wounds, skin softening, scars, acne, pores, elasticity or keloids, or for treating facial spasms, blepharospasm, torticollis, blepharospasm, cervical dystonia, central pharyngeal dystonia, spasmodic dysphonia, migraine, anal pruritus, or hyperhidrosis, comprising administering a therapeutically effective amount of the botulinum toxin composition of any one of claim 1, 2, or 12 to an individual.

15. The method of claim 14, wherein a reduced diffusion effect occurs with two or more repeated administrations.
